# EUROPEAN PATENT APPLICATION

(11) **EP 1 728 518 A1**
(43) Date of publication of application: **06.12.2006**
(21) Application number: 06011096.2
(22) Date of filing: 23.09.1999
(51) Int. Cl.: A61K 39/395, G01N 33/574, C12Q 1/68, C07K 16/30

(54) **Method of diagnosing monitoring, staging, imaging and treating gynecologic cancers and testicular cancer**

(30) Priority: 23.09.1998 US 101522 P
(62) Divisional of application: 99948349.8
(71) Applicant: Diadexus, Inc., South San Francisco, CA 94080 (US)
(72) Inventor: Ali, Shujath M., Santa Clara CA 95051 (US); Cafferkey, Robert, San Jose CA 95134 (US)
(74) Representative: Peebles, Katrina

(57) **Abstract**

The present invention provides a method of diagnosing, monitoring, staging, imaging and treating gynecologic cancers and testicular cancer.

## Description

### FIELD OF THE INVENTION

This invention relates, in part, to newly developed assays for detecting, diagnosing, monitoring, staging, prognosticating, imaging and treating cancers, particularly gynecologic cancers including ovarian, mammary, endometrial, uterine and cervical cancer, and testicular cancer.

### BACKGROUND OF THE INVENTION

In women, gynecologic cancers account for more than one-fourth of the malignancies.

Of the gynecologic cancers, breast cancer is the most common. According to the Women's Cancer Network, 1 out of every 8 women in the United States is at risk of developing breast cancer, and 1 out of every 28 women are at risk of dying from breast cancer. Approximately 77% of women diagnosed with breast cancer are over the age of 50. However, breast cancer is the leading cause of death in women between the ages of 40 and 55.

Carcinoma of the ovary is another very common gynecologic cancer. Approximately one in 70 women will develop ovarian cancer during her lifetime. An estimated 14,500 deaths in 1995 resulted from ovarian cancer. It causes more deaths than any other cancer of the female reproductive system. Ovarian cancer often does not cause any noticeable symptoms. Some possible warning signals, however, are an enlarged abdomen due to an accumulation of fluid or vague digestive disturbances (discomfort, gas or distention) in women over 40; rarely there will be abnormal vaginal bleeding. Periodic, complete pelvic examinations are important; a Pap test does not detect ovarian cancer. Annual pelvic exams are recommended for women over 40.

Also common in women is endometrial cancer or carcinoma of the lining of the uterus. According to the Women's Cancer Center endometrial cancer accounts for approximately 13% of all malignancies in women. There are about 34,000 cases of endometrial cancer diagnosed in the United States each year.

Uterine sarcoma is another type of uterine malignancy much more rare as compared to other gynecologic cancers. In uterine sarcoma, malignant cells start growing in the muscles or other supporting tissues of the uterus. Sarcoma of the uterus is different from cancer of the endometrium, a disease in which cancer cells start growing in the lining of the uterus. This uterine cancer usually begins after menopause. Women who have received therapy with high-dose X-rays (external beam radiation therapy) to their pelvis are at a higher risk to develop sarcoma of the uterus. These X-rays are sometimes given to women to stop bleeding from the uterus.

Cancer of the cervix, another common kind of cancer in women, is a disease in which cancer (malignant) cells are found in the tissues of the cervix. Cancer of the cervix usually grows slowly over a period of time. Before cancer cells are found on the cervix, the tissues of the cervix undergo changes in which abnormal cells begin to appear (known as dysplasia). A Pap smear will usually identify these cells. Later, cancer cells begin to grow and spread more deeply into the cervix and to surrounding areas. Since there are usually no symptoms associated with cancer of the cervix, accurate diagnostic tests are critical. The first of these is a Pap smear, which is done by using a piece of cotton, a brush, or a small wooden stick to gently scrape the outside of the cervix in order to pick up cells. If cells that are not normal are found, the doctor will remove a sample of tissue (this procedure is called a biopsy) from the cervix and examine it microscopically for cancer cells. A patient may need to go to the hospital if a larger, cone-shaped biopsy specimen (conization) is required. The prognosis (chance of recovery) and choice of treatment depend on the stage of the cancer (whether it is just in the cervix or has spread to other places) and the patient's general health.

The lifetime probability of developing testicular cancer is 0.2% for an American white male. The cause of testicular cancer is unknown, however, it is associated with both congenital and acquired factors. From a treatment standpoint, testicular cancer is divided into two major categories, nonseminomas and seminomas. In the commonly used staging system for nonseminomas, a stage A lesion is confined to the testis; in stage B there is regional lymph node involvement in the retroperitoneum; and in stage C, there is distant metastasis. For seminomas, a stage I lesion is confined to the testis; in stage II, the lesion has spread to the retroperitoneal lymph nodes; and in stage III, the lesion has supradiaphragmatic nodal or visceral involvement.

The most common symptom of testicular cancer is painless enlargement of the testis. Acute testicular pain resulting from intertesticular hemorrhage occurs in about 10% of cases. Patients are often asymptomatic upon presentation but about 10% may exhibit back pain, cough or lower extremity edema. A testicular mass or diffuse enlargement of the testis can be detected by physical examination in most cases. Several biochemical markers are used for the diagnosis and treatment of this disease including human chorionic gonadotropin (hCG), alpha-fetoprotein and LDH. However, an incorrect diagnosis is made initially in up to 25% of patients with testicular tumors. Accordingly, improved methods for diagnosing testicular cancer are needed.

Procedures used for detecting, diagnosing, monitoring, staging, and prognosticating these cancers are of critical importance to the outcome of the patient. Patients diagnosed early generally have a much greater five-year survival rate as compared to the survival rate for patients diagnosed with distant metastasized cancer. New diagnostic methods which are more sensitive and specific for detecting early cancers are clearly needed.

Cancer patients are closely monitored following initial therapy and during adjuvant therapy to determine response to therapy and to detect persistent or recurrent disease or metastasis. Thus, there is also clearly a need for cancer markers which are more sensitive and specific in detecting cancer recurrence.

Another important step in managing cancer is to determine the stage of the patient's disease. Stage determination has potential prognostic value and provides criteria for designing optimal therapy. Generally, pathological staging of cancer is preferable over clinical staging because the former gives a more accurate prognosis. However, clinical staging would be preferred were it at least as accurate as pathological staging because it does not depend on an invasive procedure to obtain tissue for pathological evaluation. Staging of cancer would be improved by detecting new markers in cells, tissues or bodily fluids which could differentiate between different stages of invasion.

In the present invention, methods are provided for detecting, diagnosing, monitoring, staging, prognosticating, imaging and treating gynecologic cancers or testicular cancer via a Cancer Specific Gene (CSG). The CSG refers, among other things, to native protein expressed by the gene comprising the polynucleotide sequence of SEQ ID NO:1. The amino acid sequence of a polypeptide encoded by SEQ ID NO:1 is depicted herein as SEQ ID NO:2. In the alternative, what is meant by the CSG as used herein, means the native mRNA encoded by the gene comprising the polynucleotide sequence of SEQ ID NO:1 or levels of the gene comprising the polynucleotide sequence of SEQ ID NO:1.

Other objects, features, advantages and aspects of the present invention will become apparent to those of skill in the art from the following description. It should be understood, however, that the following description and the specific examples, while indicating preferred embodiments of the invention are given by way of illustration only. Various changes and modifications within the spirit and scope of the disclosed invention will become readily apparent to those skilled in the art from reading the following description and from reading the other parts of the present disclosure.

### SUMMARY OF THE INVENTION

Toward these ends, and others, it is an object of the present invention to provide a method for diagnosing the presence of gynecologic cancers or testicular cancer by analyzing for changes in levels of CSG in cells, tissues or bodily fluids compared with levels of CSG in preferably the same cells, tissues, or bodily fluid type of a normal human control, wherein a change in levels of CSG in the patient versus the normal human control is associated with a gynecologic cancer or testicular cancer.

Further provided is a method of diagnosing metastatic cancer in a patient having a gynecologic cancer or testicular which is not known to have metastasized by identifying a human patient suspected of having a gynecologic cancer or testicular cancer that has metastasized; analyzing a sample of cells, tissues, or bodily fluid from such patient for CSG; comparing the CSG levels in such cells, tissues, or bodily fluid with levels of CSG in preferably the same cells, tissues, or bodily fluid type of a normal human control, wherein an increase in CSG levels in the patient versus the normal human control is associated with a gynecologic cancer or a testicular cancer which has metastasized.

Also provided by the invention is a method of staging gynecologic cancers or testicular cancer in a human which has such cancer by identifying a human patient having such cancer; analyzing a sample of cells, tissues, or bodily fluid from such patient for CSG; comparing CSG levels in such cells, tissues, or bodily fluid with levels of CSG in preferably the same cells, tissues, or bodily fluid type of a normal human control sample, wherein an increase in CSG levels in the patient versus the normal human control is associated with a cancer which is progressing and a decrease in the levels of CSG is associated with a cancer which is regressing or in remission.

Further provided is a method of monitoring gynecologic cancers or testicular cancer in a human having such cancer for the onset of metastasis. The method comprises identifying a human patient having such cancer that is not known to have metastasized; periodically analyzing a sample of cells, tissues, or bodily fluid from such patient for CSG; comparing the CSG levels in such cells, tissue, or bodily fluid with levels of CSG in preferably the same cells, tissues, or bodily fluid type of a normal human control sample, wherein an increase in CSG levels in the patient versus the normal human control is associated with a cancer which has metastasized.

Further provided is a method of monitoring the change in stage of a gynecologic cancer or testicular cancer in a human having such cancer by looking at levels of CSG in a human having such cancer. The method comprises identifying a human patient having such cancer; periodically analyzing a sample of cells, tissues, or bodily fluid from such patient for CSG; comparing the CSG levels in such cells, tissue, or bodily fluid with levels of CSG in preferably the same cells, tissues, or bodily fluid type of a normal human control sample, wherein an increase in CSG levels in the patient versus the normal human control is associated with a cancer which is progressing and a decrease in the levels of CSG is associated with a cancer which is regressing or in remission.

Further provided are antibodies against CSG or fragments of such antibodies which can be used to detect or image localization of CSG in a patient for the purpose of detecting or diagnosing a disease or condition. Such antibodies can be polyclonal, monoclonal, or omniclonal or prepared by molecular biology techniques. The term "antibody", as used herein and throughout the instant specification is also meant to include aptamers and single-stranded oligonucleotides such as those derived from an *in vitro* evolution protocol referred to as SELEX and well known to those skilled in the art. Antibodies can be labeled with a variety of detectable labels including, but not limited to, radioisotopes and paramagnetic metals. These antibodies or fragments thereof can also be used as therapeutic agents in the treatment of diseases characterized by expression of a CSG. In therapeutic applications, the antibody can be used without or with derivatization to a cytotoxic agent such as a radioisotope, enzyme, toxin, drug or a prodrug.

Other objects, features, advantages and aspects of the present invention will become apparent to those of skill in the art from the following description. It should be understood, however, that the following description and the specific examples, while indicating preferred embodiments of the invention, are given by way of illustration only. Various changes and modifications within the spirit and scope of the disclosed invention will become readily apparent to those skilled in the art from reading the following description and from reading the other parts of the present disclosure.

### DETAILED DESCRIPTION OF THE INVENTION

The present invention relates to diagnostic assays and methods, both quantitative and qualitative for detecting, diagnosing, monitoring, staging and prognosticating cancers by comparing levels of CSG with those of CSG in a normal human control. What is meant by levels of CSG as used herein, means levels of the native protein expressed by the gene comprising the polynucleotide sequence of SEQ ID NO:1. The amino acid sequence of a polypeptide encoded by SEQ ID NO:1 is depicted herein as SEQ ID NO:2. In the alternative, what is meant by levels of CSG as used herein, means levels of the native mRNA encoded by the gene comprising the polynucleotide sequence of SEQ ID NO:1 or levels of the gene comprising the polynucleotide sequence of SEQ ID NO:1. Such levels are preferably measured in at least one of, cells, tissues and/or bodily fluids, including determination of normal and abnormal levels. Thus, for instance, a diagnostic assay in accordance with the invention for diagnosing over-expression of CSG protein compared to normal control bodily fluids, cells, or tissue samples may be used to diagnose the presence of cancers, including gynecologic cancers such as breast, ovarian, uterine, endometrial and cervical cancer, and testicular cancer.

All the methods of the present invention may optionally include measuring the levels of other cancer markers as well as CSG. Other cancer markers, in addition to CSG, useful in the present invention will depend on the cancer being tested and are known to those of skill in the art.

### Diagnostic Assays

The present invention provides methods for diagnosing the presence of gynecologic cancers or testicular cancer by analyzing for changes in levels of CSG in cells, tissues or bodily fluids compared with levels of CSG in cells, tissues or bodily fluids of preferably the same type from a normal human control, wherein an increase in levels of CSG in the patient versus the normal human control is associated with the presence of a gynecologic cancer such as ovarian, breast, uterine, endometrial or cervical cancer, or testicular cancer.

Without limiting the instant invention, typically, for a quantitative diagnostic assay a positive result indicating the patient being tested has cancer is one in which cells, tissues or bodily fluid levels of the cancer marker, such as CSG, are at least two times higher, and most preferably are at least five times higher, than in preferably the same cells, tissues or bodily fluid of a normal human control.

The present invention also provides a method of diagnosing metastatic gynecologic cancers or testicular cancer in a patient having such cancer which has not yet metastasized for the onset of metastasis. In the method of the present invention, a human cancer patient suspected of having a gynecologic cancer or testicular cancer which may have metastasized (but which was not previously known to have metastasized) is identified. This is accomplished by a variety of means known to those of skill in the art.

In the present invention, determining the presence of CSG levels in cells, tissues or bodily fluid, is particularly useful for discriminating between a gynecologic cancer or testicular cancer which has not metastasized and a gynecologic cancer or testicular cancer which has metastasized. Existing techniques have difficulty discriminating between gynecologic cancers or testicular cancer which have metastasized and gynecologic cancers or testicular cancer which have not metastasized. However, proper treatment selection is often dependent upon such knowledge.

In the present invention, the cancer marker levels measured in such cells, tissues or bodily fluid is CSG, and are compared with levels of CSG in preferably the same cells, tissue or bodily fluid type of a normal human control. That is, if the cancer marker being observed is CSG in serum, this level is preferably compared with the level of CSG in serum of a normal human patient. An increase in the CSG in the patient versus the normal human control is associated with a gynecologic cancer or testicular cancer which has metastasized.

Without limiting the instant invention, typically, for a quantitative diagnostic assay a positive result indicating the cancer in the patient being tested or monitored has metastasized is one in which cells, tissues or bodily fluid levels of the cancer marker, such as CSG, are at least two times higher, and most preferably are at least five times higher, than in preferably the same cells, tissues or bodily fluid of a normal patient.

Normal human control as used herein includes a human patient without cancer and/or non cancerous samples from the patient; in the methods for diagnosing or monitoring for metastasis, normal human control may also include samples from a human patient that is determined by reliable methods to have a gynecologic cancer or testicular cancer which has not metastasized such as samples from the same patient prior to metastasis.

### Staging

The invention also provides a method of staging gynecologic cancers or testicular cancer in a human patient. The method comprises identifying a human patient having such cancer and analyzing a sample of cells, tissues or bodily fluid from such human patient for CSG. CSG levels in such cells, tissues or bodily fluid are then compared with levels of CSG in preferably the same cells, tissues or bodily fluid type of a normal human control sample, wherein an increase in CSG levels in the human patient versus the normal human control is associated with a cancer which is progressing and a decrease in the levels of CSG is associated with a cancer which is regressing or in remission.

### Monitoring

Further provided is a method of monitoring gynecologic cancers or testicular cancer in a human having such cancer for the onset of metastasis. The method comprises identifying a human patient having such cancer that is not known to have metastasized; periodically analyzing a sample of cells, tissues or bodily fluid from such human patient for CSG; comparing the CSG levels in such cells, tissues or bodily fluid with levels of CSG in preferably the same cells, tissues or bodily fluid type of a normal human control sample, wherein an increase in CSG levels in the human patient versus the normal human control is associated with a cancer which has metastasized.

Further provided by this invention is a method of monitoring the change in stage of gynecologic cancers or testicular cancer in a human having such cancer. The method comprises identifying a human patient having such cancer; periodically analyzing a sample of cells, tissues or bodily fluid from such human patient for CSG; comparing the CSG levels in such cells, tissues or bodily fluid with levels of CSG in preferably the same cells, tissues or bodily fluid type of a normal human control sample, wherein an increase in CSG levels in the human patient versus the normal human control is associated with a cancer which is progressing in stage and a decrease in the levels of CSG is associated with a cancer which is regressing in stage or in remission.

Monitoring such patient for onset of metastasis is periodic and preferably done on a quarterly basis. However, this may be more or less frequent depending on the cancer, the particular patient, and the stage of the cancer.

### Assay Techniques

Assay techniques that can be used to determine levels of gene expression (including protein levels), such as CSG of the present invention, in a sample derived from a patient are well known to those of skill in the art. Such assay methods include, without limitation, radioimmunoassays, reverse transcriptase PCR (RT-PCR) assays, immunohistochemistry assays, *in situ* hybridization assays, competitive-binding assays, Western Blot analyses, ELISA assays and proteomic approaches: two-dimensional gel electrophoresis (2D electrophoresis) and non-gel based approaches such as mass spectrometry or protein interaction profiling. Among these, ELISAs are frequently preferred to diagnose a gene's expressed protein in biological fluids.

An ELISA assay initially comprises preparing an antibody, if not readily available from a commercial source, specific to CSG, preferably a monoclonal antibody. In addition a reporter antibody generally is prepared which binds specifically to CSG. The reporter antibody is attached to a detectable reagent such as radioactive, fluorescent or enzymatic reagent, for example horseradish peroxidase enzyme or alkaline phosphatase.

To carry out the ELISA, antibody specific to CSG is incubated on a solid support, e.g. a polystyrene dish, that binds the antibody. Any free protein binding sites on the dish are then covered by incubating with a non-specific protein such as bovine serum albumin. Next, the sample to be analyzed is incubated in the dish, during which time CSG binds to the specific antibody attached to the polystyrene dish. Unbound sample is washed out with buffer. A reporter antibody specifically directed to CSG and linked to horseradish peroxidase is placed in the dish resulting in binding of the reporter antibody to any monoclonal antibody bound to CSG. Unattached reporter antibody is then washed out. Reagents for peroxidase activity, including a colorimetric substrate are then added to the dish. Immobilized peroxidase, linked to CSG antibodies, produces a colored reaction product. The amount of color developed in a given time period is proportional to the amount of CSG protein present in the sample. Quantitative results typically are obtained by reference to a standard curve.

A competition assay may be employed wherein antibodies specific to CSG attached to a solid support and labeled CSG and a sample derived from the host are passed over the solid support and the amount of label detected attached to the solid support can be correlated to a quantity of CSG in the sample.

Nucleic acid methods may be used to detect CSG mRNA as a marker for ovarian and testicular cancer. Polymerase chain reaction (PCR) and other nucleic acid methods, such as ligase chain reaction (LCR) and nucleic acid sequence based amplification (NASABA), can be used to detect malignant cells for diagnosis and monitoring of various malignancies. For example, reverse-transcriptase PCR (RT-PCR) is a powerful technique which can be used to detect the presence of a specific mRNA population in a complex mixture of thousands of other mRNA species. In RT-PCR, an mRNA species is first reverse transcribed to complementary DNA (cDNA) with use of the enzyme reverse transcriptase; the cDNA is then amplified as in a standard PCR reaction. RT-PCR. can thus reveal by amplification the presence of a single species of mRNA. Accordingly, if the mRNA is highly specific for the cell that produces it, RT-PCR can be used to identify the presence of a specific type of cell.

Hybridization to clones or oligonucleotides arrayed on a solid support (i.e. gridding) can be used to both detect the expression of and quantitate the level of expression of that gene. In this approach, a cDNA encoding the CSG gene is fixed to a substrate. The substrate may be of any suitable type including but not limited to glass, nitrocellulose, nylon or plastic. At least a portion of the DNA encoding the CSG gene is attached to the substrate and then incubated with the analyte, which may be RNA or a complementary DNA (cDNA) copy of the RNA, isolated from the tissue of interest. Hybridization between the substrate bound DNA and the analyte can be detected and quantitated by several means including but not limited to radioactive labeling or fluorescence labeling of the analyte or a secondary molecule designed to detect the hybrid. Quantitation of the level of gene expression can be done by comparison of the intensity of the signal from the analyte compared with that determined from known standards. The standards can be obtained by *in vitro* transcription of the target gene, quantitating the yield, and then using that material to generate a standard curve.

Of the proteomic approaches, 2D electrophoresis is a technique well known to those in the art. Isolation of individual proteins from a sample such as serum is accomplished using sequential separation of proteins by different characteristics usually on polyacrylamide gels. First, proteins are separated by size using an electric current. The current acts uniformly on all proteins, so smaller proteins move farther on the gel than larger proteins. The second dimension applies a current perpendicular to the first and separates proteins not on the basis of size but on the specific electric charge carried by each protein. Since no two proteins with different sequences are identical on the basis of both size and charge, the result of a 2D separation is a square gel in which each protein occupies a unique spot. Analysis of the spots with chemical or antibody probes, or subsequent protein microsequencing can reveal the relative abundance of a given protein and the identity of the proteins in the sample.

The above tests can be carried out on samples derived from a variety of patients' cells, bodily fluids and/or tissue extracts (homogenates or solubilized tissue) such as from tissue biopsy and autopsy material. Bodily fluids useful in the present invention include blood, urine, saliva or any other bodily secretion or derivative thereof. Blood can include whole blood, plasma, serum or any derivative of blood.

### In Vivo Antibody Use

Antibodies against CSG can also be used *in vivo* in patients suspected of suffering from gynecologic cancers such as ovarian, breast, endometrial, uterine or cervical cancer or testicular cancer. Specifically, antibodies against a CSG can be injected into a patient suspected of having a gynecologic cancer or testicular cancer for diagnostic and/or therapeutic purposes. The use of antibodies for *in vivo* diagnosis is well known in the art. For example, antibody-chelators labeled with Indium-111 have been described for use in the radioimmunoscintographic imaging of carcinoembryonic antigen expressing tumors (Sumerdon et al. Nucl. Med. Biol. 1990 17:297-254). In particular, these antibody-chelators have been used in detecting tumors in patients suspected of having recurrent colorectal cancer (Griffin et al. J. Clin. Onc. 1991 9:631-640). Antibodies with paramagnetic ions as labels for use in magnetic resonance imaging have also been described (Lauffer, R.B. Magnetic Resonance in Medicine 1991 22:339-392). Antibodies directed against CSGs can be used in a similar manner. Labeled antibodies against a CSG can be injected into patients suspected of having a gynecologic cancer or testicular cancer for the purpose of diagnosing or staging of the disease status of the patient. The label used will be selected in accordance with the imaging modality to be used. For example, radioactive labels such as Indium-111, Technetium-99m or Iodine-131 can be used for planar scans or single photon emission computed tomography (SPECT). Positron emitting labels such as Fluorine-19 can be used in positron emission tomography. Paramagnetic ions such as Gadlinium (III) or Manganese (II) can be used in magnetic resonance imaging (MRI). Localization of the label permits determination of the spread of the cancer. The amount of label within an organ or tissue also allows determination of the presence or absence of cancer in that organ or tissue.

For patients diagnosed with a gynecologic cancer or testicular, injection of an antibody against a CSG can also have a therapeutic benefit. The antibody may exert its therapeutic effect alone. Alternatively, the antibody is conjugated to a cytotoxic agent such as a drug, toxin or radionuclide to enhance its therapeutic effect. Drug monoclonal antibodies have been described in the art for example by Garnett and Baldwin, Cancer Research 1986 46:2407-2412. The use of toxins conjugated to monoclonal antibodies for the therapy of various cancers has also been described by Pastan et al. Cell 1986 47:641-648. Yttrium-90 labeled monoclonal antibodies have been described for maximization of dose delivered to the tumor while limiting toxicity to normal tissues (Goodwin and Meares Cancer Supplement 1997 80:2675-2680). Other cytotoxic radionuclides including, but not limited to Copper-67, Iodine-131 and Rhenium-186 can also be used for labeling of antibodies against CSGs.

Antibodies which can be used in these *in vivo* methods include both polyclonal, monoclonal or omniclonal antibodies and antibodies prepared via molecular biology techniques. Antibody fragments and aptamers and single-stranded oligonucleotides such as those derived from an *in vitro* evolution protocol referred to as SELEX and well known to those skilled in the art can also be used.

### EXAMPLES

The present invention is further described by the following examples. These examples are provided solely to illustrate the invention by reference to specific embodiments. This exemplification, while illustrating certain aspects of the invention, does not portray the limitations or circumscribe the scope of the disclosed invention.

### Example 1

Identification of the CSG of SEQ ID NO:1 was carried out by a systematic analysis of data in the LIFESEQ database available from Incyte Pharmaceuticals, Palo Alto, CA, using the subsetting Search Tool of the LIEESEQ database available from Incyte Pharmaceuticals, Palo Alto, CA. Search Tools include: Library Comparison which compares one library to one other library and allows the identification of clones expressed in tumor and absent or expressed at a lower level in normal tissue; Subsetting which is similar to library comparison but allows the identification of clones expressed in a pool of libraries and absent or expressed at a lower level in a second pool of libraries; and Transcript Imaging which lists all of the clones in a single library or a pool of libraries based on abundance. Individual clones are examined using Electronic Northerns to determine the tissue sources of their component ESTs.

The following example was carried out using standard techniques, which are well known and routine to those of skill in the art, except where otherwise described in detail. Routine molecular biology techniques of the following example can be carried out as described in standard laboratory manuals, such as Sambrook et al., MOLECULAR CLONING: A LABORATORY MANUAL, 2nd Ed.; Cold Spring Harbor Laboratory Press, Cold Spring Harbor, N.Y. (1989).

### Example 2: Relative Quantitation of Gene Expression

Real-Time quantitative PCR with fluorescent Taqman probes is a quantitation detection system utilizing the 5'- 3' nuclease activity of Taq DNA polymerase. The method uses an internal fluorescent oligonucleotide probe (Taqman) labeled with a 5' reporter dye and a downstream, 3' quencher dye. During PCR, the 5'-3' nuclease activity of Taq DNA polymerase releases the reporter, whose fluorescence can then be detected by the laser detector of the Model 7700 Sequence Detection System (PE Applied Biosystems, Foster City, CA, USA).

Amplification of an endogenous control was used to standardize the amount of sample RNA added to the reaction and normalize for Reverse Transcriptase (RT) efficiency. Either cyclophilin, glyceraldehyde-3-phosphate dehydrogenase (GAPDH) or 18S ribosomal RNA (rRNA) was used as this endogenous control. To calculate relative quantitation between all the samples studied, the target RNA levels for one sample were used as the basis for comparative results (calibrator). Quantitation relative to the "calibrator" is obtained using the standard curve method or the comparative method (User Bulletin #2: ABI PRISM 7700 Sequence Detection System).

The tissue distribution and the level of the target gene in normal and cancer tissue were evaluated. Total RNA was extracted from normal tissues, cancer tissues and from cancers and the corresponding matched adjacent tissues. Subsequently, first strand cDNA was prepared with reverse transcriptase and the polymerase chain reaction was done using primers and Taqman probe specific to each target gene. The results are analyzed using the ABI PRISM 7700 Sequence Detector. The absolute numbers are relative levels of expression of the target gene in a particular tissue compared to the calibrator tissue.

### Measurement of SEQ ID NO:1; Clone ID 1450626; Gene ID 236019 ("Pro104")

The absolute numbers depicted in Table 1 are relative levels of expression of Pro104 in 12 normal different tissues. All the values are compared to normal colon (calibrator). These RNA samples are commercially available pools, originated by pooling samples of a particular tissue from different individuals.

**Table 1: Relative Levels of Pro104 Expression in Pooled Samples**

| **Tissue** | **NORMAL** |
|---|---|
| Colon Ascending | 1.0 |
| Endometrium | 0 |
| Kidney | 0 |
| Liver | 0 |
| Ovary | 0 |
| Pancreas | 0 |
| Prostate | 1.0 |
| Small Intestine | 0 |
| Spleen | 0 |
| Stomach | 0 |
| Testis | 100 |
| Uterus | 0 |

The relative levels of expression in Table 1 show that Pro104 mRNA is not expressed in normal ovary. Its expression is, however, higher (100) in testis compared with all the other normal tissues analyzed. Testis, with a relative expression level of 100, prostate (1.0), and colon (1.0) are the only tissues expressing mRNA for Pro104.

The absolute numbers in Table 1 were obtained analyzing pools of samples of a particular tissue from different individuals. They cannot be compared to the absolute,numbers originated from RNA obtained from tissue samples of a single individual in Table 2.

The absolute numbers depicted in Table 2 are relative levels of expression of Pro104 in 70 pairs of matching and 30 unmatched tissue samples. All the values are compared to normal colon(calibrator). A matching pair is formed by mRNA from the cancer sample for a particular tissue and mRNA from the normal adjacent sample for that same tissue (NAT) from the same individual. In cancers (for example, ovary) where it was not possible to obtain normal adjacent samples from the same individual, samples from a different normal individual were analyzed.

**Table 2: Relative Levels of Pro104 Expression in Individual Samples**

| **Sample ID** | **Cancer Type** | **Tissue** | **Cancer** | **Matching or NAT** |
|---|---|---|---|---|
| OVR1037O/1038 | Papillary serous adenocarcinoma | Ovary 1 | 187.3 | 0.6 |
| OVR1305/13060 | Papillary serous adenocarcinoma | Ovary 2 | 56.3 | 0 |
| OVR9410C360 | Endometrioid Adenocarcinoma | Ovary 3 | 0.9 | |
| OVR14604A1C | Cancer | Ovary 4 | 9.1 | |
| OVR1157O | Papillary serous adenocarcinoma | Ovary 5 | 10.26 | |
| OVR1040O | Papillary serous adenocarcinoma | Ovary 6 | 169.3 | |
| OVR14471A1B | Adenocarcinoma | Ovary 7 | 0.3 | |
| OVR1028O | Ovarian carcinoma | Ovary 8 | 3.2 | |
| OVR1118O | Small cell carcinoma | Ovary 9 | 0.05 | |
| OVR773O | Metastatic papillary serous adenocarcinoma | Ovary 10 | 1.3 | |
| OVR14603AlD | Adenocarcinoma | Ovary 11 | 3.5 | |
| OVR1005O | Papillary serous & endometrioid | Ovary 12 | 15 | |
| OVR9702C018G | Normal cystic | Ovary 13 | | 1.2 |
| OVR206I | Normal left atrophic, small cystic | Ovary 14 | | 0.2 |
| OVR9702C020G | Normal, multiple ovarian cysts | Ovary 15 | | 0.1 |
| OVR9702C025G | Normal, hemorrhage CL cyst | Ovary 16 | | 0.4 |
| OVR9701C035G | Normal | Ovary 17 | | 0 |
| OVR9701C040G | Normal, benign follicular cysts | Ovary 18 | | 0 |
| OVR9701C050G | Normal, multiple ovarian cysts | Ovary 19 | | 0 |
| OVR9701C109R | Normal | Ovary 20 | | 0 |
| OVR9702C004G | Normal | Ovary 21 | | 0 |
| OVR9702C007R | Normal | Ovary 22 | | 0 |
| OVR9701C087R | Normal, small follicle cysts | Ovary 23 | | 0 |
| OVR9411C109 | Normal | Ovary 24 | | 0 |
| OVR9701C177a | Normal, cystic | Ovary 25 | | 0 |
| OVR9701c179A | Normal | Ovary 26 | | 0.05 |
| OVR1461O | Serous cystadenofibroma | Ovary 27 | | 0 |
| OVR14638A1C | Right follicular cyst | Ovary 28 | | 0 |
| OVR9411C057R | Benign large endometriotic cyst | Ovary 29 | | 0 |
| UTR1358O/13590 | Tumor/NAT | Uterus 1 | 0 | 0 |
| UTR1417O/1418O | Malignant tumor/NAT | Uterus 2 | 4.51 | 0.5 |
| UTR233U96/234U96 | Adenocarcinoma/NAT | Uterus 3 | 4.29 | 1.67 |
| UTR850U/851U | Stage 1 endometrial cancer/NAT | Uterus 4 | 2.4 | 0 |
| END10479B/10479D | Disease/NAT | Endometrium 1 | 15.2 | 0 |
| END9705A125A/126 | Endometrial carcinoma/NAT | Endometrium 2 | 4.67 | 0.53 |
| END9704C281A/282 | Endometrial adenocarcinoma/NAT | Endometrium 3 | 2.54 | 0.36 |
| END14863A1A/A2A | Moderately differ. Endometr. carcin./NAT | Endometrium 4 | 0.32 | 0.31 |
| END9709C056 A/C055a | Adenocarcinoma of endometrium/NAT | Endometrium 5 | 0.41 | 0.29 |
| END | Invasive endometrial | Endometrium 6 | 0.09 | 1.36 |
| END8911A/8911D | Disease/NAT | Endometrium 7 | 0 | 0.7 |
| END8963A/8963B | Disease/NAT | Endometrium 8 | 0 | 0 |
| END9807A080A/081 | Endometrial carcinoma/NAT | Endometrium 9 | 0.15 | 0.09 |
| END9705A0/9705A0 | | Endometrium 10 | 0 | 0 |
| CVXVNM00/VNM00 | | Cervix 1 | 0.1 | 0 |
| CVXVNM00/VNM00 | | Cervix 2 | 0.4 | 0 |
| CVXIND000/IND000 | | Cervix 3 | 47.3 | 12.4 |
| CVXIND000/IND000 | | Cervix 4 | 0.5 | 0.3 |
| MAMM826I/828I | Infiltrating ductal adenocarcinoma/NAT | Mammary 1 | 0.1 | 0 |
| MAMM473P/475P | Disease/NAT | Mammary 2 | 0 | 0 |
| MAMM9706A066G/67 | Disease/NAT | Mammary 3 | 0.08 | 0.17 |
| MAMM9703B011d/b | Infiltrating ductal adenocarcinoma/NAT | Mammary 4 | 1.76 | 0.26 |
| MAMM0008603M | Infiltrating ductal breast cancer | Mammary 5 | 0 | |
| MAMM9703A004B | Normal | Mammary 6 | | 0 |
| STM4004864/4864 | | Stomach 1 | 0.43 | 0.47 |
| STM4004509/4509 | | Stomach 2 | 0.7 | 51.94 |
| STM4004154/4154 | | Stomach 3 | 0.21 | 0.11 |
| STM4004317/4317 | | Stomach 4 | 0 | 0.12 |
| CLN4004535A2/B2 | Asc. Colon, adenocarcinoma, stage D | Colon 1 | 0 | 0 |
| CLN | Rectum, Stage A | Colon 2 | 0 | 0 |
| CLN | Rectosigmoid, stage A | Colon 3 | 0 | 0 |
| CLN9612B006/B005 | Asc. Colon, cecum, adenocarcinoma | Colon 4 | 0 | 0 |
| CLN703C091R/92RB | Rectosigmoid, stage T1 | Colon 5 | 0 | 0 |
| LNG976Q/477Q | Right Lung carcinoma | Lung 1 | 0 | 0 |
| LNG605L/606L | Right Lung carcinoma | Lung 2 | 0 | 0 |
| LNG750C/751C | Metastatic osteogenic sarcoma | Lung 3 | 0 | 0 |
| PANC714L/715L | Villous adenoma | Pancreas 1 | 0 | 0 |
| PANC824P/825P | Cystic adenoma | Pancreas 2 | 0 | 0 |
| PANC10343a/10343b | | Pancreas 3 | 0.52 | 1.15 |
| PANC776P/777P | | Pancreas 4 | 0.11 | 0.08 |
| PANC921O/922O | | Pancreas 5 | 109.81 | 0 |
| LIV150A/151A | Hepatocellular carcinoma | Liver 1 | 0 | 0 |
| LIV942A/943A | Hepatoblastoma | Liver 2 | 0 | 0 |
| LIV12742B/12742C | | Liver 3 | 0.06 | 0 |
| BLD327K/328K | Papillary transitional cell carcinoma/NAT | Bladder 1 | 0 | 0 |
| BLD467K/468K | Consistent w/ high grade urothelial carcin. | Bladder 2 | 0 | 0 |
| BLD1496K/1497K | | Bladder 3 | 0.6 | 0.2 |
| BLD1721K/1722K | | Bladder 4 | 0 | 0 |
| KID1064D/1065D | Tumor/NAT | Kidney 1 | 0 | 0 |
| KID1079D/1080D | Tumor/NAT | Kidney 2 | 0.3 | 0.4 |
| KID1097D/1098D | Tumor/NAT | Kidney 3 | 0 | 0 |
| KID1263D/1264D | Tumor/NAT | Kidney 4 | 0 | 0 |
| KID512D/513D | Palpable renal cell carcinoma | Kidney 5 | 0.1 | 0.13 |
| KID689D/690D | Kidney carcinoma | Kidney 6 | 1.82 | 0 |
| KID988D/989D | Renal cell carcinoma | Kidney 7 | 1.7 | 0.2 |
| KID1024D/1025D | | Kidney 8 | 0 | 0 |
| KID1183D/1184D | | Kidney 9 | 0.7 | 0.1 |
| KID1229D/1230D | | Kidney 10 | 0 | 9.2 |
| PRO1291B/1292B | Adenocarcinoma/NAT | Prostate 1 | 11.9 | 0.2 |
| PRO209B/210B | Adenocarcinoma of the prostate/NAT | Prostate 2 | 0 | 0 |
| PRO1222B/1223B | Adenocarcinoma/NAT | Prostate 3 | 19.9 | 3.2 |
| PRO1293B/1294B | Adenocarcinoma/NAT | Prostate 4 | 0.7 | 4.4 |
| PRO650B/651B | Adenocarcinoma/NAT | Prostate 5 | 1.34 | 0.15 |
| PR0694B/695B | | Prostate 6 | 0 | 0 |
| PRO780B/781B | Adenocarcinoma/NAT | Prostate 7 | 0.2 | 0.8 |
| PRO845B/846B | Adenocarcinoma/NAT | Prostate 8 | 0.2 | 0 |
| PRO902B/903B | | Prostate 9 | 0 | 0 |
| PRO916B/917B | Tumor/NAT | Prostate 10 | 0 | 0 |
| PRO1012B/1013B | | Prostate 11 | 3.27 | 2.74 |
| PRO139B/140B | | Prostate 12 | 0.19 | 0.01 |
| TST239X/240X | Tumor/NAT | Testis 1 | 1.66 | 8.19 |

| | | | | |
|---|---|---|---|---|
| 0= Negative | | | | |

In the analysis of all samples, the higher levels of expression were in tumor ovary (median 6.3), showing a high degree of specificity for ovarian cancer. Expression of Pro104 was also found in other cancers of the female reproductive tissues including the cervix, endometrium, uterus, and breast. The median level in these tissues was 3.2). Of all the samples (other than the female reproductive tissues) analyzed, only one sample (the cancer sample Pancreas 5 with 109.81) showed an expression comparable to the mRNA expression in ovarian cancer. The median for the expression of Pro104 for all other cancer tissues was zero.

The level of mRNA expression in cancer samples and from the isogenic normal adjacent tissue from the same individual or normal adjacent tissue from a different individual were also compared. This comparison provides an indication of specificity for the cancer stage (e.g. higher levels of mRNA expression in the cancer sample compared to the normal adjacent). Table 2 shows an induction of Pro104 expression in 2 ovary cancer tissues compared with their respective normal adjacent (ovary samples # 1, 2) and, the induction of Pro104 in 10 unmatched cancer tissues (ovary samples #3 through 12) compared with 17 normal adjacent tissues (ovary samples 13 through 29). There was induction of Pro104 expression in the cancer tissue for 100% of the ovary samples tested (total of 2 matching and 10 unmatched ovary samples). However, 71% of the normal adjacent tissue samples were negative, and 29% (5 of 17) showed a low level of Pro104 expression of 0.05 - 1.2 normalized units.

Altogether, the absence of expression in 71% of the normal adjacent tissues (with negligible expression in the remaining 29% normal adjacents), plus the higher level of mRNA expression in 100% of the ovary tumor samples tested is indicative of Pro104 being a diagnostic marker for ovarian cancer. Expression of this marker in the cervix, endometrium, uterus, and mammary cancers, is indicative of P104 also being a diagnostic marker in other gynecologic cancers.

Based on the amino acid composition as depicted in SEQ ID NO:2, Pro104 is a serine protease that shares 37% homology with human hepsin at the nucleotide level, and 31% homology at the level of amino acids. When aligned with several other serine proteases such as kallikrein-1, kallikrein-2, prostate specific antigen, prostasin, protease M, and hepsin, Pro104 shares all the conserved amino acid motifs that are characteristic of all other serine proteases. For example, Pro104 contains a highly conserved RIVGG (SEQ ID NO:3) sequence. Cleavage between arginine (R) and isoleucine (I) results in the activation of proteases that contain the motif (Kurachi et al. Hepsin. Methods in Enzymology, Vol. 244, pp. 100-114 (1994)). The active protease domain of Pro104 therefore consists of 273 amino acids. Pro104 also contains the conserved histidine, aspartic acid, and serine residues that form a catalytic triad for enzymatic function.

## Claims

1. A method for diagnosing the presence of a gynecologic cancer or testicular cancer in a patient comprising:
(a) measuring levels of CSG in cells, tissues or bodily fluids in a patient; and
(b) comparing the measured levels of CSG with levels of CSG in cells, tissues or bodily fluids from a normal human control, wherein a change in measured levels of CSG in said patient versus normal human control is associated with the presence of a gynecologic cancer or testicular cancer.

2. A method of diagnosing metastases of a gynecologic cancer or testicular cancer in a patient comprising:
(a) identifying a patient having a selected cancer that is not known to have metastasized;
(b) measuring CSG levels in a sample of cells, tissues, or bodily fluid from said patient; and
(c) comparing the measured CSG levels with levels of CSG in cells, tissue, or bodily fluid of a normal human control, wherein an increase in measured CSG levels in the patient versus the normal human control is associated with a cancer which has metastasized.

3. A method of staging a gynecologic cancer or testicular cancer in a patient having a gynecologic cancer or testicular cancer comprising:
(a) identifying a patient having the cancer;
(b) measuring CSG levels in a sample of cells, tissue, or bodily fluid from said patient; and
(c) comparing measured CSG levels with levels of CSG in cells, tissues, or bodily fluid of a normal human control sample, wherein an increase in measured CSG levels in said patient versus the normal human control is associated with a cancer which is progressing and a decrease in the measured CSG levels is associated with a cancer which is regressing or in remission.

4. A method of monitoring a gynecologic cancer or testicular cancer in a patient for the onset of metastasis comprising:
(a) identifying a patient having a gynecologic cancer or testicular cancer that is not known to have metastasized;
(b) periodically measuring levels of CSG in samples of cells, tissues, or bodily fluid from said patient for CSG; and
(c) comparing the periodically measured CSG levels with levels of CSG in cells, tissues, or bodily fluid of a normal human control, wherein an increase in any one of the periodically measured CSG levels in the patient versus the normal human control is associated with a cancer which has metastasized.

5. A method of monitoring the change in stage of a gynecologic cancer or testicular cancer in a patient comprising:
(a) identifying a patient having a gynecologic cancer or testicular cancer;
(b) periodically measuring levels of CSG in cells, tissues, or bodily fluid from said patient for CSG; and
(c) comparing the periodically measured CSG levels with levels of CSG in cells, tissues, or bodily fluid of a normal human control, wherein an increase in any one of the periodically measured CSG levels in the patient versus the normal human control is associated with a cancer which is progressing in stage and a decrease is associated with a cancer which is regressing in stage or in remission.

6. The method of claim 1, 2, 3, 4 or 5 wherein the CSG comprises SEQ ID NO:1.

7. An antibody against a CSG wherein said CSG comprises SEQ ID NO:1.

8. A method of imaging a gynecologic cancer or testicular cancer in a patient comprising administering to the patient an antibody of claim 7.

9. The method of claim 8 wherein said antibody is labeled with paramagnetic ions or a radioisotope.

10. A method of treating a gynecologic cancer or testicular cancer in a patient comprising administering to the patient an antibody of claim 7.

11. The method of claim 10 wherein the antibody is conjugated to a cytotoxic agent.


SEQUENCE LISTING

<110> Shujath, Ali M.
      Cafferkey, Robert
      DIADEXUS LLC

<120> Method of Diagnosing, Monitoring, Staging, Imaging and
      Treating Gynecologic Cancers and Testicular Cancer

<130> DEX-0044

<140>
<141>

<150> 60/101,522
<151> 1998-09-23

<160> 3

<170> PatentIn Ver. 2.0

<210> 1
<211> 1081
<212> DNA
<213> Homo sapiens

<400> 1
aggaggcaga gggggcgtca ggccgcggga gaggaggcca tgggcgcgcg cggggcgctg 60
ctgctggcgc tgctgctggc tcgggctgga ctcaggaagc cggagtcgca ggaggcggcg 120
cccttatcag gaccatgcgg ccgacgggtc atcacgtcgc gcatcgtggg tggagaggac 180
gccgaactcg ggcgttggcc gtggcagggg agcctgcgcc tgtgggattc ccacgtatgc 240
ggagtgagcc tgctcagcca ccgctgggca ctcacggcgg cgcactgctt tgaaacctat 300
agtgacctta gtgatccctc cgggtggatg gtccagtttg gccagctgac ttccatgcca 360
tccttctgga gcctgcaggc ctactacacc cgttacttcg tatcgaatat ctatctgagc 420
cctcgctacc tggggaattc accctatgac attgccttgg tgaagctgtc tgcacctgtc 480
acctacacta aacacatcca gcccatctgt ctccaggcct ccacatttga gtttgagaac 540
cggacagact gctgggtgac tggctggggg tacatcaaag aggatgaggc actgccatct 600
ccccacaccc tccaggaagt tcaggtcgcc atcataaaca actctatgtg caaccacctc 660
ttcctcaagt acagtttccg caaggacatc tttggagaca tggtttgtgc tggcaatgcc 720
caaggcggga aggatgcctg cttcggtgac tcaggtggac ccttggcctg taacaagaat 780
ggactgtggt atcagattgg agtcgtgagc tggggagtgg gctgtggtcg gcccaatcgg 840
cccggtgtct acaccaatat cagccaccac tttgagtgga tccagaagct gatggcccag 900
agtggcatgt cccagccaga cccctcctgg ccactactct ttttccctct tctctgggct 960
ctcccactcc tggggccggt ctgagcctac ctgagcccat gcagcctggg gccactgcca 1020
agtcaggccc tggttctctt ctgtcttgtt tggtaataaa cacattccag ttgatgcctg 1080
c                                                                 1081

<210> 2

<211> 327
<212> PRT
<213> Homo sapiens

<400> 2
Arg Arg Gln Arg Gly Arg Gln Ala Ala Gly Glu Glu Ala Met Gly Ala
1               5                   10                  15

Arg Gly Ala Leu Leu Leu Ala Leu Leu Leu Ala Arg Ala Gly Leu Arg
            20                  25                  30

Lys Pro Glu Ser Gln Glu Ala Ala Pro Leu Ser Gly Pro Cys Gly Arg
        35                  40                  45

Arg Val Ile Thr Ser Arg Ile Val Gly Gly Glu Asp Ala Glu Leu Gly
    50                  55                  60

Arg Trp Pro Trp Gln Gly Ser Leu Arg Leu Trp Asp Ser His Val Cys
65                  70                  75                  80

Gly Val Ser Leu Leu Ser His Arg Trp Ala Leu Thr Ala Ala His Cys
                85                  90                  95

Phe Glu Thr Tyr Ser Asp Leu Ser Asp Pro Ser Gly Trp Met Val Gln
            100                 105                 110

Phe Gly Gln Leu Thr Ser Met Pro Ser Phe Trp Ser Leu Gln Ala Tyr
        115                 120                 125

Tyr Thr Arg Tyr Phe Val Ser Asn Ile Tyr Leu Ser Pro Arg Tyr Leu
    130                 135                 140

Gly Asn Ser Pro Tyr Asp Ile Ala Leu Val Lys Leu Ser Ala Pro Val
145                 150                 155                 160

Thr Tyr Thr Lys His Ile Gln Pro Ile Cys Leu Gln Ala Ser Thr Phe
                165                 170                 175

Glu Phe Glu Asn Arg Thr Asp Cys Trp Val Thr Gly Trp Gly Tyr Ile
            180                 185                 190

Lys Glu Asp Glu Ala Leu Pro Ser Pro His Thr Leu Gln Glu Val Gln
        195                 200                 205

Val Ala Ile Ile Asn Asn Ser Met Cys Asn His Leu Phe Leu Lys Tyr
    210                 215                 220

Ser Phe Arg Lys Asp Ile Phe Gly Asp Met Val Cys Ala Gly Asn Ala

225                 230                 235                 240

Gln Gly Gly Lys Asp Ala Cys Phe Gly Asp Ser Gly Gly Pro Leu Ala
                245                 250                 255

Cys Asn Lys Asn Gly Leu Trp Tyr Gln Ile Gly Val Val Ser Trp Gly
            260                 265                 270

Val Gly Cys Gly Arg Pro Asn Arg Pro Gly Val Tyr Thr Asn Ile Ser
        275                 280                 285

His His Phe Glu Trp Ile Gln Lys Leu Met Ala Gln Ser Gly Met Ser
    290                 295                 300

Gln Pro Asp Pro Ser Trp Pro Leu Leu Phe Phe Pro Leu Leu Trp Ala
305                 310                 315                 320

Leu Pro Leu Leu Gly Pro Val
                325

<210> 3
<211> 5
<212> PRT
<213> Homo sapiens

<400> 3
Arg Ile Val Gly Gly
1               5